# EUROPEAN PATENT SPECIFICATION

(11) **EP 4 659 795 B1**
(45) Date of publication and mention of the grant of the patent: **05.08.2026**
(21) Application number: 24179675.4
(22) Date of filing: 03.06.2024
(51) Int. Cl.: A61N 1/04, A61N 1/36, A61B 5/00, A61H 23/00, A61N 2/00, A61N 5/00, A61B 5/024, A61B 5/0533, A61H 23/02, A61N 2/06, A61N 5/06

(54) **WRIST-WORN INDEPENDENT 3-POINT ACUPUNCTURE STIMULATOR**
AM HANDGELENK GETRAGENER UNABHÄNGIGER AKUPUNKTURSTIMULATOR MIT DREI PUNKTEN
STIMULATEUR D'ACUPUNCTURE INDÉPENDANT À TROIS POINTS PORTÉ AU POIGNET

(43) Date of publication of application: 10.12.2025
(73) Proprietor: Kostopoulos, Larry, Toronto ON M8X 2T4 (CA)
(72) Inventor: Kostopoulos, Larry, Toronto ON M8X 2T4 (CA)
(74) Representative: Reich, Jochen

(56) References cited:
- AU-A1- 2019 364 532
- CN-A- 111 529 923
- CN-A- 116 785 158
- CN-U- 212 700 093

## Description

### BACKGROUND

### Field of the Invention

The present invention relates generally to the field of wrist-worn equipment, and more particularly to a wrist-worn device that can independently stimulate any one, any two or all three acupuncture points P6, H7 and Lu9.

CN 212 700 093 U shows a three-acupoint microcurrent stimulation health care device for a plant nervous system, which comprises a shell, an indicator light and an operation button which are arranged on the front side of the shell, a first electrode, a second electrode and a third electrode which are arranged on the back side of the shell, and a lithium battery and a control panel which are arranged inside the shell, wherein the lithium battery, the indicator light, the operation button and the first electrode, the second electrode and the third electrode are respectively electrically connected with the control panel.

CN 111 529 923 A shows a wearable device for providing electronic impulses comprises an acquisition module for acquiring physiological information; the control module is connected with the acquisition module and used for selecting a corresponding output mode according to the acquired physiological information and controlling the output of the electronic pulse; the electrode plate is arranged on one side, which is in contact with a human body, of the wearable device, and is connected with the control module and used for outputting electronic pulses; wherein the output pattern is determined by any one or any combination of frequency, pulse width, intensity, duration of the electronic pulses.

### Description of the Problem Solved

Nausea, anxiety and stress are three common problems that numerous people experience or suffer from. It is well-known in the art of acupuncture that there are three acupuncture points on the palmar (inside) side of the wrist. These three points are called Buddha's Triangle, and consist of point P6 or Pericardium 6, H7 or Heart 7 (sometimes HT7), and Lung 9 (Lu9).

Stimulating point P6 helps unbind the chest, regulate the heart and calm the mind; it is also very effective in combating nausea and vomiting.

Stimulating point H7 helps to calm the mind as well as help with insomnia, talking during sleep, poor memory, mania-depression, dementia, sadness, fear, disorientation and grief as well as providing relief for heart-related emotional issues.

Stimulating Lu9 helps control the nervous system and relieve acute stress by helping with breathing. Shallow breathing is not just a stress response; it becomes a habit that feeds stress. Shallow breathing also lowers the amount of lymphocytes, a type of white blood cell that helps to defend the body from invading organisms as well as countless other negative health effects. Stimulation of these acupuncture points can be accomplished by electrical pulses or currents, magnetic fields and directed light.

It would be advantageous to have a wrist-worn stimulator that could stimulate any one of these points independently or any combination of them simultaneously including all three at the same time.

### SUMMARY OF THE INVENTION

The invention is defined by the appended claims. The present disclosure relates to a method and device for controlling nausea, anxiety and stress. The device includes a wristwatch-like housing, electrical circuitry for generating electrical impulses of which low-frequency direct current (DC) electrical pulses are preferred, a metal ground plate, three adjustable metal contact points, indicator lights, function buttons on the housing, and a strap for securing the housing to the wrist. There may also be a screen display for visualizations of different functions. An internal processor controls all device functions and can optionally communicate via USB or short-range communications with a telephone App. The method allows simultaneous application of a pulse voltage output, haptic output, light emission, or magnetic field emission to acupuncture points H7, Lu9 and P6 as well as individual application to the points separately or in any combination. Another embodiment of the disclosure simply uses three permanent magnets positioned over the acupuncture points H7, Lu9 and P6 when worn.

In an alternate embodiment of the apparatus, the device monitors the user's Heart Rate Variability (HRV) and Galvanic Skin Response (GSR) to determine the user's stress level, and if warranted, provides feedback to the user using sound frequencies driving haptic actuators located over the P6, H7 Lu9 acupuncture points to help de-stress the user.

### DESCRIPTION OF THE FIGURES

Attention is now directed to several drawings that illustrate features of the present disclosure.
**Fig. 1** shows an embodiment of the present disclosure that resembles a wristwatch.
**Figs. 2A-2B** show an alternate embodiment of the present disclosure top and bottom.
**Fig. 3** is a block diagram of an electrical circuit that can independently provide electro-stimulation to one or more of the three wrist acupuncture points.
**Fig. 4** shows magnetic stimulation.
**Fig. 5** shows light stimulation.
**Fig. 6** is a flow chart for feedback stress turn-on.
**Fig. 7** shows a permanent magnet embodiment of the present disclosure.

Several figures and illustrations have been provided to aid in understanding the present disclosure.

### DESCRIPTION OF THE PREFERRED EMBODIMENTS

The present disclosure relates to a device for controlling nausea, anxiety and stress.

The device includes a wristwatch-like housing, electrical circuitry for generating electrical impulses of which low-frequency, direct current (DC) electrical impulses are preferred, a metal ground plate, three adjustable metal contact points, indicator lights, function buttons on the housing, and a strap for securing the housing to the wrist. There may also be a screen display for visualizations of different functions.

In the following description, a direct current (DC) pulse or DC square-wave pulse is a pulse whose time signature does not cross zero volts (always positive with respect to a ground reference electrode). Thus, a 1 volt DC pulse pulses from zero when low to 1 volt peak when high.

The word "approximately" in reference to any value being discussed or claimed means that the actual value (which can vary with temperature or other ambient or electronic conditions) is close to the specified value (within plus or minus 1%).

In various embodiments of the disclosure, stimulation may be alternatively or cumulatively produced using outputs such as directed light such as that from low-level lasers or light-emitting diodes (LEDs), electrical current, soundwaves, or by magnetic fields produced by magnets such as small electromagnets.

Feedback may be used in an open-or closed-loop control system to initiate or modulate the stimulation. For example, photoplethysmography (PPG) may be used to detect changes in heart rate (HR) and heart rate variability (HRV) which are key indicators of a person's stress level. A person's stress level may also be detected using Galvanic Skin Response (GSR) sensor whereby, on a person's skin, changes in electrical conductivity are detected in response to sweat gland activity. Upon the GSR sensor detecting increased conductivity, the wrist-worn acupuncture stimulation device may be activated for a set period of time to apply a stimulation output. The stimulation output may be applying a plurality of direct current pulses via the three supply electrodes, directed light such as that from low-level lasers or LEDs, haptic output such as soundwaves, or magnetic output such as magnetic fields produced by magnets such as small electromagnets. In an alternative embodiment, a person's stress level may also be detected using cortisol sensing via a cortisol aptasensor or functional equivalent. Upon the cortisol aptasensor detecting increased cortisol, the wrist-worn acupuncture stimulation device may be activated for a set period of time applying an output as mentioned above or a combination of the outputs mentioned above. When negative HRV changes are detected (indicating stress), the device can automatically activate for a set period of time, such as from one to five minute(s) of treatment time.

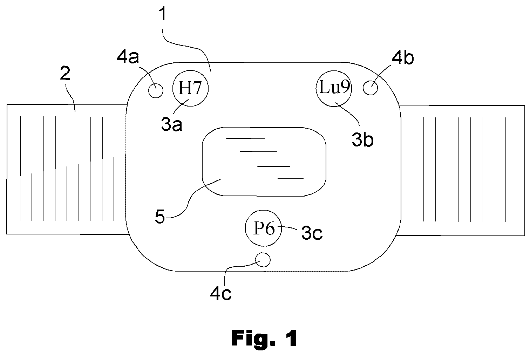

Fig. 1 shows an embodiment of the present disclosure. A wristwatch-like case **1** has a strap or band **2.** The case in this embodiment has three buttons with indicators for the three acupuncture points. Button **3a** activates stimulation to point H7 lighting indicator **4a.** Button **3b** activates stimulation to point LU1 lighting indicator **4b.** Button **4c** activates stimulation to point P6 lighting indicator **4c.** The indicators **4a, 4b** and **4c** can be small LEDs or other types of lights. The embodiment of Fig. 1 can also be equipped with a display screen that can optionally be a touch screen. This screen can be used to select combinations of the points being stimulated, and it can be used to control the device such as setting a timer.

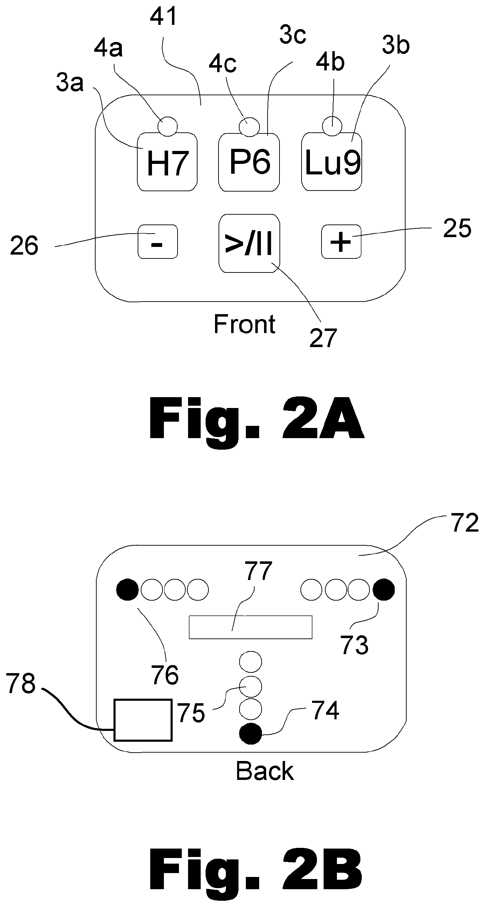

Fig. 2A shows a top view of an alternate embodiment of the device. This embodiment is an electro-stimulator supplying low-level direct current (DC) square-wave signal to the acupuncture point. The buttons **3a, 3b** and **3c** have been made larger and easier to read, while the indicators **4a, 4b** and **4c** have been moved closer to the corresponding button. The functions of these three buttons and indicators are the same as that of the embodiment of Fig. 1. There are also three additional buttons plus **25,** minus **26** and play/pause/cancel **27** marked respectively+, - and >/II- Touching plus **25** or minus **26** causes the magnitude of the applied output voltage level, luminosity, magnetic field strength, and/or haptic amplitude, to be increased or decreased. Touching the play/pause/cancel button **27** starts the stimulation, pauses the stimulation, or cancels the selected stimulation. A combination of the stimulated points can be selected by touching more than one button simultaneously.

Fig. 2B shows a bottom view of the embodiment of Fig. 2A. As the embodiment shown in Figs 2A and 2B is one configured to output current, three electrode sets are shown **73, 74** and **76.** For each electrode set, four point positions are shown. The black dot indicates the selected position, while the white (open) dots **75** are the unselected positions. Changing the selected electrode position can be done manually in some embodiments, or electronically in other embodiments using additional buttons and an optional view screen. The optional view screen allows the user to see the current test being administered, and see and adjust the selected electrode position. A return path for current or ground electrode **77** can be seen in Fig. 2B. Alternatively, the bottom surface of the device of Fig. 2B can take the form of a metal plate. The ground return electrode **77** should be positioned so that current from the activated electrode **73, 74** or **76,** or combinations of them, passes through the acupuncture point. Note that in embodiments whereby the output is in the form of directed light such as that from low-level lasers or LEDs, haptic output such as soundwaves, or magnetic output such as magnetic fields produced by magnets such as small electromagnets, corresponding stimulation emitters will be positioned in the same location as that of electrodes **73, 74** and **76.** Specifically, where the stimulation output is to be directed light, electrodes **73, 74** and **76** will be replaced or supplemented by light emitters such as laser emitters or LEDs. Where the stimulation output is to be a haptic output, electrodes **73, 74** and **76** will be replaced or supplemented by sound or vibration emitters. Where the stimulation output is to be magnetic output, electrodes **73, 74** and **76** will be replaced or supplemented by electromagnets.

In some embodiments of the disclosure, the wrist-worn device shown in Figs. 2A-2B or Fig. 1 can communicate with a user's hand-held telephone via short-range radio (like Bluetooth (TM)) or other short-range communication techniques like magnetic induction. In this case, a complete program or App can be run on the telephone that shows the stimulation running, shows the selected electrodes, contains timers and the like and contains stored sequences of treatment for more complex application of stimulus. The touch screen on the telephone can be used to change internal parameters in the wrist-worn unit such as the location of the chosen electrode or stimulation emitter position for example.

In an alternative embodiment, a person's stress level may also be detected using Galvanic Skin Response (GSR) sensor whereby, on a person's skin, changes in electrical conductivity are detected in response to sweat gland activity. In this embodiment, the conductivity of a user's skin may be measured using one of the electrodes 73, 74, or 76 and the ground electrode 77. A detected electrical resistance between one of the electrodes 73, 74, or 76 and the ground electrode 77 will yield a measured skin conductivity. Changes in the measured skin conductivity over time can be calculated to determine the user's stress level. Specifically, upon the GSR sensor detecting increased conductivity, the wrist-worn acupuncture stimulation device may be activated for a set period of time. The activation may result in an output that comprises applying a plurality of direct current pulses via the three supply electrodes **73, 74,** and/or **76,** applying directed light from light emitters positioned in the same position as supply electrodes **73, 74,** and/or **76,** applying vibratory haptic or sound stimulation by sound or vibration emitters positioned in the same position as supply electrodes **73, 74,** and/or **76,** and/or applying magnetic stimulation by electromagnets positioned in the same position as supply electrodes **73, 74,** and/or **76.**

In an alternative embodiment, a person's stress level may also be detected using cortisol sensing via a cortisol aptasensor or functional equivalent. As shown in Figure 2B, the cortisol aptasensor 78 may be in the form of a cortisol-detecting strip in contact with the skin of the user. Upon the cortisol aptasensor 78 detecting increased cortisol, the wrist-worn acupuncture stimulation device may be activated for a set period of time applying an electrical, haptic, magnetic, or light output, as mentioned above.

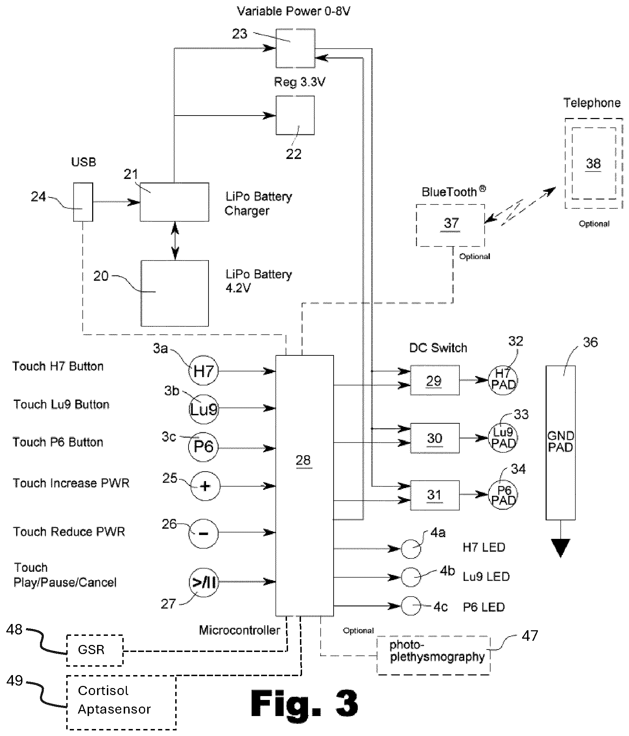

Fig. 3 shows a block diagram of the embodiment of Figs. 2A-2B. A battery, preferably a Lithium-Polymer (LiPo) **20** supplies power for the unit. This battery typically provides 4.2 volts.

While a battery can be changed periodically like a watch battery, it is preferable to have one that can be recharged using methods known in the art. LiPo batteries can be recharged using a micro-USB socket **24** and charging control circuitry **21.**

Small micro-circuits such as microcontrollers usually run on 3.3 - 3.7 volts. Using a 4.2 V LiPo battery **20** thus requires a 3.3 volt regulator **22** that runs all circuitry except providing power for the actual applied stimulation output pulses (normal power wiring not shown).

In an embodiment where the stimulation emitters are electrodes 73, 74, and 76, the output may be in the form of applied current pulses that are preferably square waves of approximately 2.5 Hz with a peak voltage of between approximately 1.0 and 8.8 volts. For safety and power conservation, the pulses need to be limited to approximately 600 uA or less. While 2.5 Hz square waves are preferred, other frequencies or repetition rates and/or pulse shapes and duty cycles are within the scope of the present disclosure

For example, the pulse repetition rate can be between approximately 2.0 Hz and 3.0 Hz A variable power supply **23** supplies these pulses. This may be a single integrated circuit (IC) chip or discrete components. The variable power supply **23** can contain a voltage doubler circuit feeding a controllable output voltage. The voltage can be assigned by the microcontroller **28** and communicated to the variable power supply **23** by serial or parallel data or by analog voltage.

The microcontroller or micro-processor **28** can be any standard micro computing device known in art. For ease of manufacturing, the micro-controller preferably includes internal random access memory (RAM) and read-only memory (ROM). It can be mask programmed at manufacture, or it can be done with downloaded updates via the optional short-range communication **37** like Bluetooth (TM) or from the USB port **24.** In addition to program updates, the USB port can be used to perform diagnostics and the like. The optional short- range communication **37** can be used to supply an app in the telephone **38** with system status including, but not limited to, battery charge, current pulse voltage, current pulse frequency or repetition rate, current pulse shape and the like.

The microcontroller **28** receives input from the six keys **3a, 3b, 3c, 25, 26, 27.** This communication can use direct connection or a matrix type arrangement used in key pads. The microcontroller **28** can optionally communicate mono- or bi-directionally with the USB port **24** and/or the optionally short-range communication **37.**

The microcontroller **28** has two sets of outputs: drives for the LEDs and on/off commands a set of switches that control the applied stimulation output. The LEDs **4a, 4b, 4c** can be brightness modulated to indicate the applied pulse intensity (applied voltage resulting in current through the acupuncture point. The LED's are brightness modulated by allowing more or less current to flow through them. The circuitry for this may be internal to the microcontroller **28,** or it may be external in the form of an LED control chip (not shown).

A set of electronic switches **29, 30, 31** provide off and on control to the three acupuncture pads H7 **32**, Lu9 **33** and P6 **34**. In the embodiment shown in Fig. 3, the microcontroller **28** simply turns the switches **29, 30, 31** off or on, while sending the selected voltage to the variable power supply **23.**

This method is the easiest and cheapest to manufacture; however, it has the disadvantage that all electrodes or stimulation emitters being used simultaneously (such as for example H7 combined with P6) have the same voltage.

While this is normally acceptable, if, in a more complex stimulation routine, it is desired to have the different acupuncture points activated with different intensities (voltages), or even with different pulses, the variable power supply **23** can have three separate independently controllable voltage outputs, one for each electrode. The microcontroller **28,** in this case can set each voltage separately at the variable power supply **23** and/or control the pulse shape with the switches **29, 30, 31.** A serial message from the microcontroller **28** to the variable power supply 23 could contain three separate fields; alternatively, three outputs (wires) from the microcontroller **28** can run to the variable power supply **23.**

In an embodiment where the stimulation emitters are electrodes 73, 74, and 76, the square wave or pulse can be generated in one of two ways: 1) it can be generated by having the microcontroller **28** simply turn the variable power supply **23** off and on, or turn the selected switch(es) **29, 30, 31** on and off at the correct frequency, or 2) the variable power supply **23** can itself contain an oscillator that produces the waveform. The easiest way is to simply have the microcontroller **28** create the waveform. This reduces the complexity of the variable power supply **23.** It should be noted that all currents return to the variable power supply through the ground pad **36.** Generally, this can be the same ground as the 3.3 V system ground; however, it may also be an isolated ground (ground wiring not shown in Fig. 3).

Fig. 3 also shows an optional photoplethysmography device **47** that measures the heart rate variability (HRV). This can be used for closed-loop control to be described. A Galvanic Skin Response (GSR) sensor 48 and/or a cortisol aptasensor 49 may also be connected to the microcontroller 28 to enable detection of stress change from GSR changes or cortisol changes, respectively.

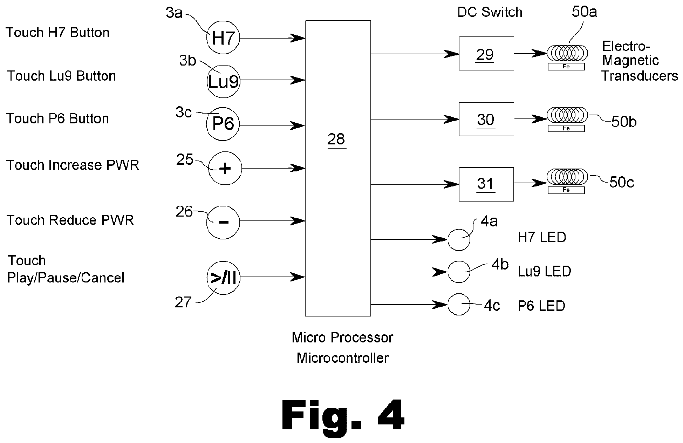

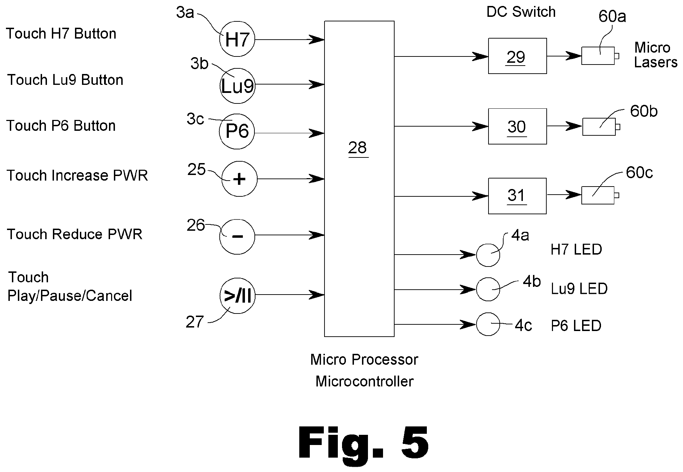

Fig. 4 shows magnetic stimulation, while Fig. 5 shows light stimulation. In Fig. 4, small electromagnetic transducer coils **50a, 50b** and **50c** replace the electrodes; in Fig. 5 very small lasers or LEDs **60a, 60b** and **50c** replace the electrodes.

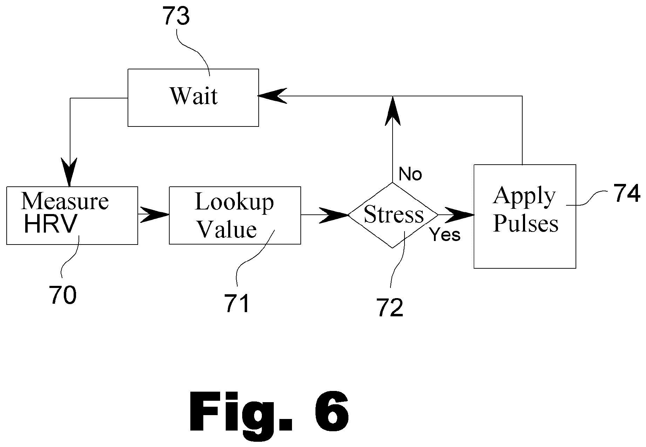

As previously stated, feedback may be used in an open-or closed-loop control system to initiate or modulate the stimulation. An example feedback loop is shown in Fig. 6. As stated, photoplethysmography (PPG) may be used to detect changes in heart rate (HR) and heart rate variability (HRV) which are key indicators of a person's stress level. The HRV (and optionally the HR) are measured **70** by the device. The HRV level is looked up **71** in a table when negative HRV changes are detected (indicating some stress). If the table indicates stress **72** the device can automatically activate for a set period of time **73,** such as from one to five minute(s) of treatment time. When there is no stress, HRV can be checked again; however, a much longer wait **73** can be executed to save battery power. Also, the table lookup **71** is optional, the device can declare stress whenever a negative HRV is measured.

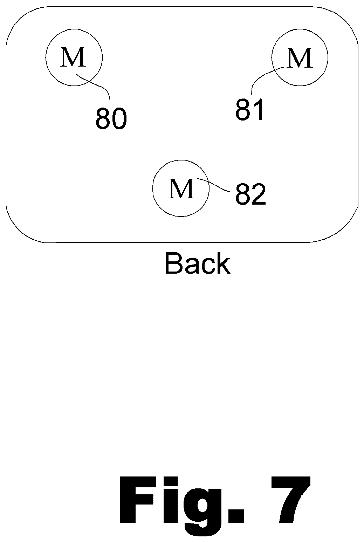

An alternate embodiment shown in Fig. 7 uses three permanent magnets to simultaneously stimulate the three points of Buddha's Triangle, H7, Lu9 and P6. Small cylindrical magnets are preferred. The flat end of the cylinder magnet encounters the acupuncture point.

### Typical Electrical Parameters

Human skin resistance can run from around 1000 Ohms to around 100 k Ohms depending on contact area, moisture and condition of the skin.

During stimulation mode the preferred pulses have the following output parameters at a skin impedance of 100 k Ohms:
Voltage 0 - 8.8 V Frequency 2.5 Hz DC Square wave (a square wave that is entirely of positive or negative voltage with respect to ground).

The pulse current varies with the intensity setting:
At the maximum intensity setting (applying 8.8 volts): the following currents result:
100 k Ohms impedance gives 85-88 uA.
10 k Ohms impedance would give 880 uA (unless current is further limited by the circuitry or power supply capability to 600 uA or less).

It is desirable to limit current for safety and for battery conservation. The preferred maximum current is 600 uA or less.

Minimum intensity (about 1 V applied) is between 6 - 8 uA.

### Sound Frequency to De-stress

In an alternate embodiment of the apparatus, the device monitors the user's heart Rate Variability (HRV) or Galvanic Skin Response (GSR) or both to determine the user's stress level, and if warranted, provides feedback to the user using sound frequencies driving haptic actuators located over the P6, H7 Lu9 acupuncture points to help de-stress the user.

This embodiment incorporates features such as compact design, battery-powered (one day per charging cycle is typical). The device is wrist-worn with sensors positioned to monitor heart rate variability (HRV) or and galvanic skin response (GSR) or both, and then activate feedback when threshold values are reached. There is an optional screen configured to display HRV, GSR, frequency, intensity, points being stimulated, and the like.

Three well-defined positions on the wrist (P6, H7 Lu9), previously discussed, are optimally located to provide feedback to the user via sound waves driven by haptic actuators. These points can be stimulated individually, simultaneously, or in any combination.

The optimum stimulation frequency lies between 100 20-400 Hertz but can be determined by testing. The frequency chosen may be tuned for an individual user. Intensity and frequencies are variable and can be adjusted by the user or automatically. The user can override the triggers generated by the thresholds of
HRV and GSR, and receive self-administered treatment outside of a stress event.

In general, the device will be worn all day. However, the user can choose when to wear it based on experience with the device.

The device of this embodiment can also communicate HRV and GSR data wirelessly with a smartphone, and can maintain and transmit to an App on the smartphone a recorded chronological log of HRV and of GSR events over a particular time period such a daytime working hours and/or events that occur during sleep hours (many times when a user awakens during the night and begins to worry about some detail in their life, job or relationship).

## Claims

1. A wrist-worn acupuncture stimulation device comprising:
a watch-like case (1) having a top side and bottom side;
a wrist strap or band (2) attachable to the watch-like case constructed to allow the case to be worn on a human wrist;
three stimulation emitters (73, 74, 76) mounted on the bottom side of the case, the three stimulation emitters electrically connected to a variable voltage power supply (23) and located in positions corresponding to the H7, Lu9 and P6 acupuncture points, the three stimulation emitters configured to stimulate the H7, Lu9 and P6 acupuncture points;
**characterised in that** the stimulation device further comprises
a galvanic skin response (GSR) sensor (48) configured to measure changes in electrical conductivity of a skin on the human wrist, wherein upon the GSR sensor detecting increased conductivity, the wrist-worn acupuncture stimulation device is activated for a set period of time applying stimulation to the H7, Lu9 and P6 acupuncture points via the three stimulation emitters; and
a microcontroller (28) in communication with the three stimulation emitters, wherein the microcontroller is configured to communicate bi-directionally via short-range communication with a telephone App.

2. The wrist-worn acupuncture stimulation device of claim 1, further comprising:
a ground electrode mounted on the bottom side of the case, and
wherein the three stimulation emitters are electrodes configured to emit current directly to the H7, Lu9 and P6 acupuncture points, and the ground electrode is configured to collect current from any or all the three electrodes after passing through the H7, Lu9 or P6 acupuncture points, and return said current to the variable voltage pulse power supply.

3. The wrist-worn acupuncture stimulation device of claim 1 or 2, further comprising a photoplethysmography (PPG) device configured to measure at least heart rate variability rate (HRV) and heart rate (HR).

4. The wrist-worn acupuncture stimulation device according to any one of the previous claims, wherein upon a PPG device detecting a decrease in HRV level, the wrist-worn acupuncture stimulation device is activated for a set period of time.

5. The wrist-worn acupuncture stimulation device according to any one of the previous claims, further comprising short-range communications with a mobile telephone, wherein the mobile telephone executes a stored application program utilizing said short-range communications.

6. The wrist-worn acupuncture stimulation device according to any one of the previous claims, wherein the three stimulation emitters are electrodes configured to emit current as the stimulation, light emitters configured to emit directed light as the stimulation, haptic emitters configured to emit sound or vibration as the stimulation, or electromagnets configured to emit magnetic fields as the stimulation.

7. The wrist-worn acupuncture stimulation device according to any one of the previous claims, wherein the three stimulation emitters are a combination of two or more of the following: electrodes configured to emit current as the stimulation, light emitters configured to emit directed light as the stimulation, haptic emitters configured to emit sound or vibration as the stimulation, or electromagnets configured to emit magnetic fields as the stimulation.

8. A wrist-worn acupuncture stimulation device comprising:
a watch-like case (1) having a top side and bottom side;
a wrist strap or band (2) attachable to the watch-like case constructed to allow the case to be worn on a human wrist;
three stimulation emitters (73, 74, 76) mounted on the bottom side of the case, the three stimulation emitters electrically connected to a variable voltage power supply (23) and located in positions corresponding to the H7, Lu9 and P6 acupuncture points, the three stimulation emitters configured to stimulate the H7, Lu9 and P6 acupuncture points; and
**characterised in that** the stimulation device also comprises a cortisol aptasensor (49, 78) configured to measure cortisol levels in the human wrist, wherein upon the cortisol aptasensor detecting increased cortisol, the wrist-worn acupuncture stimulation device is activated for a set period of time applying a stimulation via the three stimulation emitters.

9. The wrist-worn acupuncture stimulation device of claim 8, further comprising:
a ground electrode mounted on the bottom side of the case, and
wherein the three stimulation emitters are electrodes configured to emit current directly to the H7, Lu9 and P6 acupuncture points, and the ground electrode is configured to collect current from any or all the three electrodes after passing through the H7, Lu9 or P6 acupuncture points, and return said current to the variable voltage pulse power supply.

10. The wrist-worn acupuncture stimulation device of claim 8 or 9, further comprising a photoplethysmography (PPG) device configured to measure at least heart rate variability rate (HRV) and heart rate (HR).

11. The wrist-worn acupuncture stimulation device of claim 10, wherein upon the PPG device detecting a decrease in HRV level, the wrist-worn acupuncture stimulation device is activated for a set period of time.

12. The wrist-worn acupuncture stimulation device according to any one of claims 8 to 11, further comprising short-range communications with a mobile telephone, wherein the mobile telephone executes a stored application program utilizing said short-range communications.

13. The wrist-worn acupuncture stimulation device according to any one of claims 8 to 12, wherein the three stimulation emitters are electrodes configured to emit current as the stimulation, light emitters configured to emit directed light as the stimulation, haptic emitters configured to emit sound or vibration as the stimulation, or electromagnets configured to emit magnetic fields as the stimulation.

14. The wrist-worn acupuncture stimulation device according to any one of claims 8 to 13, wherein the three stimulation emitters are a combination of two or more of the following: electrodes configured to emit current as the stimulation, light emitters configured to emit directed light as the stimulation, haptic emitters configured to emit sound or vibration as the stimulation, or electromagnets configured to emit magnetic fields as the stimulation.

## Patentansprüche

1. Handgelenk getragenes Akupunktur-Stimulationsgerät, umfassend:einem uhrenartigen Gehäuse (1) mit einer Oberseite und einer Unterseite;
einem am uhrenartigen Gehäuse befestigbaren Armband (2), das so konstruiert ist, dass das Gehäuse am menschlichen Handgelenk getragen werden kann;
drei Stimulationsemitter (73, 74, 75), die an der Unterseite des Gehäuses angebracht sind, wobei die drei Stimulationsemitter elektrisch mit einer variablen Spannungsversorgung (23) verbunden sind und sich an Positionen befinden, die den Akupunkturpunkten H7, Lu9 und P6 entsprechen, wobei die drei Stimulationsemitter so konfiguriert sind, dass sie die Akupunkturpunkte H7, Lu9 und P6 stimulieren; **dadurch gekennzeichnet, dass** die Stimulationsvorrichtung ferner einen Galvanic-Skin-Response-Sensor (GSR-Sensor) (48) umfasst, der so konfiguriert ist, dass er Änderungen der elektrischen Leitfähigkeit der Haut am menschlichen Handgelenk misst, wobei, sobald der GSR-Sensor eine erhöhte Leitfähigkeit erfasst, die am Handgelenk getragene Akupunktur-Stimulationsvorrichtung für einen festgelegten Zeitraum aktiviert wird, um über die drei Stimulationsemitter eine Stimulation an den Akupunkturpunkten H7, Lu9 und P6 anzulegen; und
einen Mikrocontroller (28), der mit den drei Stimulationsemittern in Verbindung steht, wobei der Mikrocontroller so konfiguriert ist, dass er über eine Nahbereichskommunikation bidirektional mit einer Telefon-App kommuniziert.

2. Handgelenk getragene Akupunktur-Stimulationsgerät nach Anspruch 1, das ferner umfasst:
eine an der Unterseite des Gehäuses angebrachte Masseelektrode, und
wobei die drei Stimulationsemitter Elektroden sind, die so konfiguriert sind, dass sie Strom direkt an die Akupunkturpunkte H7, Lu9 und P6 abgeben, und die Masseelektrode so konfiguriert ist, dass sie Strom von einer oder allen drei Elektroden aufnimmt, nachdem dieser die Akupunkturpunkte H7, Lu9 oder P6 durchlaufen hat, und diesen Strom an die variable Spannungsimpuls-Stromversorgung zurückleitet.

3. Handgelenk getragene Akupunktur-Stimulationsgerät nach Anspruch 1 oder 2, das ferner eine Photoplethysmographie-Vorrichtung (PPG) umfasst, die so konfiguriert ist, dass sie mindestens die Herzfrequenzvariabilität (HRV) und die Herzfrequenz (HR) misst.

4. Handgelenk getragene Akupunkturstimulationsgerät gemäß einem der vorstehenden Ansprüche, wobei das am Handgelenk getragene Akupunkturstimulationsgerät für einen festgelegten Zeitraum aktiviert wird, sobald eine PPG-Vorrichtung einen Rückgang des HRV-Wertes feststellt.

5. Handgelenk getragene Akupunktur-Stimulationsgerät gemäß einem der vorstehenden Ansprüche, das ferner eine Kurzstreckenkommunikation mit einem Mobiltelefon umfasst, wobei das Mobiltelefon ein gespeichertes Anwendungsprogramm unter Verwendung der genannten Kurzstreckenkommunikation ausführt.

6. Handgelenk getragene Akupunktur-Stimulationsgerät gemäß einem der vorstehenden Ansprüche, wobei die drei Stimulationsemitter Elektroden sind, die so konfiguriert sind, dass sie Strom als Stimulation abgeben, Lichtemitter, die so konfiguriert sind, dass sie gerichtetes Licht als Stimulation abgeben, haptische Emitter, die so konfiguriert sind, dass sie Schall oder Vibrationen als Stimulation abgeben, oder Elektromagnete, die so konfiguriert sind, dass sie Magnetfelder als Stimulation abgeben.

7. Handgelenk getragene Akupunktur-Stimulationsgerät gemäß einem der vorstehenden Ansprüche, wobei die drei Stimulationsemitter eine Kombination aus zwei oder mehr der folgenden Elemente sind: Elektroden, die so konfiguriert sind, dass sie Strom als Stimulation abgeben, Lichtemitter, die so konfiguriert sind, dass sie gerichtetes Licht als Stimulation abgeben, haptische Emitter, die so konfiguriert sind, dass sie Schall oder Vibrationen als Stimulation abgeben, oder Elektromagnete, die so konfiguriert sind, dass sie Magnetfelder als Stimulation abgeben.

8. Handgelenk getragenes Akupunktur-Stimulationsgerät, umfassend:
ein uhrenähnliches Gehäuse (1) mit einer Oberseite und einer Unterseite;
einem am uhrenartigen Gehäuse befestigbaren Armband (2), das so konstruiert ist, dass das Gehäuse am menschlichen Handgelenk getragen werden kann;
drei Stimulationssender (73, 74, 76), die an der Unterseite des Gehäuses angebracht sind, wobei die drei Stimulationssender elektrisch mit einer variablen Spannungsversorgung (23) verbunden sind und sich an Positionen befinden, die den Akupunkturpunkten H7, Lu9 und P6 entsprechen,
wobei die drei Stimulationssender so konfiguriert sind, dass sie die Akupunkturpunkte H7, Lu9 und P6 zu stimulieren; und **dadurch gekennzeichnet, dass** die Stimulationsvorrichtung ferner einen Cortisol-Aptasensor (49, 78) umfasst, der so konfiguriert ist, dass er Cortisolspiegel am menschlichen Handgelenk misst, wobei bei Erfassung eines erhöhten Cortisolspiegels durch den Cortisol-Aptasensor die am Handgelenk getragene Akupunktur-Stimulationsvorrichtung für einen festgelegten Zeitraum aktiviert wird, um über die drei Stimulationsemitter eine Stimulation abzugeben.

9. Handgelenk getragene Akupunktur-Stimulationsvorrichtung nach Anspruch 8, ferner umfassend:
eine an der Unterseite des Gehäuses angebrachte Masseelektrode, und
wobei die drei Stimulationsemitter Elektroden sind, die so konfiguriert sind, dass sie Strom direkt an die Akupunkturpunkte H7, Lu9 und P6 abgeben, und die Masseelektrode so konfiguriert ist, dass sie Strom von einem oder allen der drei Elektroden aufnimmt, nachdem dieser die Akupunkturpunkte H7, Lu9 oder P6 durchlaufen hat, und diesen Strom an die variable Spannungsimpuls-Stromversorgung zurückleitet.

10. Handgelenk getragene Akupunktur-Stimulationsgerät nach Anspruch 8 oder 9, das ferner eine Photoplethysmographie-Vorrichtung (PPG-Vorrichtung) umfasst, die so konfiguriert ist, dass sie zumindest die Herzfrequenzvariabilität (HRV) und die Herzfrequenz (HR) misst.

11. Handgelenk getragene Akupunkturstimulationsgerät nach Anspruch 10, wobei das am Handgelenk getragene Akupunkturstimulationsgerät für einen festgelegten Zeitraum aktiviert wird, sobald die PPG-Vorrichtung einen Rückgang des HRV-Wertes feststellt.

12. Handgelenk getragene Akupunktur-Stimulationsgerät gemäß einem der Ansprüche 8 bis 11, das ferner eine Kurzstreckenkommunikation mit einem Mobiltelefon umfasst, wobei das Mobiltelefon ein gespeichertes Anwendungsprogramm unter Verwendung der genannten Kurzstreckenkommunikation ausführt.

13. Handgelenk getragene Akupunktur-Stimulationsgerät gemäß einem der Ansprüche 8 bis 12, wobei die drei Stimulationsemitter Elektroden sind, die so konfiguriert sind, dass sie Strom als Stimulation abgeben, Lichtemitter, die so konfiguriert sind, dass sie gerichtetes Licht als Stimulation abgeben, haptische Emitter, die so konfiguriert sind, dass sie Schall oder Vibration als Stimulation abgeben, oder Elektromagnete, die so konfiguriert sind, dass sie Magnetfelder als Stimulation abgeben.

14. Handgelenk getragene Akupunktur-Stimulationsgerät gemäß einem der Ansprüche 8 bis 13, wobei die drei Stimulationsemitter eine Kombination aus zwei oder mehr der folgenden Elemente sind: Elektroden, die so konfiguriert sind, dass sie Strom als Stimulation abgeben, Lichtemitter, die so konfiguriert sind, dass sie gerichtetes Licht als Stimulation abgeben, haptische Emitter, die so konfiguriert sind, dass sie Schall oder Vibrationen als Stimulation abgeben, oder Elektromagnete, die so konfiguriert sind, dass sie Magnetfelder als Stimulation abgeben.

## Revendications

1. Dispositif de stimulation par acupuncture porté au poignet, comprenant :
un boîtier de type montre (1) comportant une face supérieure et une face inférieure ;
un bracelet (2) pouvant être fixé au boîtier de type montre, conçu pour permettre le port du boîtier au poignet d'une personne ;
trois émetteurs de stimulation (73, 74, 75) montés sur la face inférieure du boîtier, les trois émetteurs de stimulation étant connectés électriquement à une alimentation à tension variable (23) et situés à des positions correspondant aux points d'acupuncture H7, Lu9 et P6, les trois émetteurs de stimulation étant configurés pour stimuler les points d'acupuncture H7, Lu9 et P6 ;
**caractérisé en ce que** le dispositif de stimulation comprend en outre un capteur de réponse galvanique de la peau (GSR) (48) configuré pour mesurer les variations de conductivité électrique de la peau du poignet humain, dans lequel, lorsque le capteur GSR détecte une augmentation de la conductivité, le dispositif de stimulation d'acupuncture porté au poignet est activé pendant une durée prédéfinie, appliquant une stimulation aux points d'acupuncture H7, Lu9 et P6 via les trois émetteurs de stimulation ; et
un microcontrôleur (28) en communication avec les trois émetteurs de stimulation, dans lequel le microcontrôleur est configuré pour communiquer de manière bidirectionnelle via une communication à courte portée avec une application téléphonique.

2. Dispositif de stimulation par acupuncture porté au poignet selon la revendication 1, comprenant en outre :
une électrode de masse montée sur la face inférieure du boîtier, et
dans lequel les trois émetteurs de stimulation sont des électrodes configurées pour émettre un courant directement vers les points d'acupuncture H7, Lu9 et P6, et l'électrode de masse est configurée pour collecter le courant provenant de l'une ou de l'ensemble des trois électrodes après son passage par les points d'acupuncture H7, Lu9 ou P6, et renvoyer ledit courant vers l'alimentation en impulsions à tension variable.

3. Dispositif de stimulation par acupuncture porté au poignet selon la revendication 1 ou 2, comprenant en outre un dispositif de photopléthysmographie (PPG) configuré pour mesurer au moins la variabilité de la fréquence cardiaque (VFC) et la fréquence cardiaque (FC).

4. Dispositif de stimulation par acupuncture porté au poignet selon l'une quelconque des revendications précédentes, dans lequel, lorsqu'un dispositif PPG détecte une diminution du niveau de HRV, le dispositif de stimulation par acupuncture porté au poignet est activé pendant une durée prédéfinie.

5. Dispositif de stimulation par acupuncture porté au poignet selon l'une quelconque des revendications précédentes, comprenant en outre une communication à courte portée avec un téléphone mobile, dans lequel le téléphone mobile exécute un programme d'application stocké utilisant ladite communication à courte portée.

6. Dispositif de stimulation par acupuncture porté au poignet selon l'une quelconque des revendications précédentes, dans lequel les trois émetteurs de stimulation sont des électrodes configurées pour émettre un courant en tant que stimulation, des émetteurs de lumière configurés pour émettre une lumière dirigée en tant que stimulation, des émetteurs d' s haptiques configurés pour émettre un son ou une vibration en tant que stimulation, ou des électroaimants configurés pour émettre des champs magnétiques en tant que stimulation.

7. Dispositif de stimulation par acupuncture porté au poignet selon l'une quelconque des revendications précédentes, dans lequel les trois émetteurs de stimulation sont une combinaison de deux ou plusieurs des éléments suivants : des électrodes configurées pour émettre un courant en tant que stimulation, des émetteurs de lumière configurés pour émettre une lumière dirigée en tant que stimulation, des émetteurs haptiques configurés pour émettre un son ou une vibration en tant que stimulation, ou des électroaimants configurés pour émettre des champs magnétiques en tant que stimulation.

8. Dispositif de stimulation par acupuncture porté au poignet comprenant :
un boîtier de type montre (1) comportant une face supérieure et une face inférieure ;
un bracelet ou une sangle (2) pouvant être fixé au boîtier de type montre, conçu pour permettre au boîtier d'être porté au poignet d'une personne ;
trois émetteurs de stimulation (73, 74, 76) montés sur la face inférieure du boîtier, les trois émetteurs de stimulation étant reliés électriquement à une alimentation à tension variable (23) et situés à des emplacements correspondant aux points d'acupuncture H7, Lu9 et P6, les trois émetteurs de stimulation étant configurés pour stimuler les points d'acupuncture H7, Lu9 et P6 ;
et **caractérisé en ce que** le dispositif de stimulation comprend également un aptasenseur de cortisol (49, 78) configuré pour mesurer les niveaux de cortisol au niveau du poignet humain, dans lequel, lorsque l'aptasenseur de cortisol détecte une augmentation du cortisol, le dispositif de stimulation d'acupuncture porté au poignet est activé pendant une durée prédéfinie, appliquant une stimulation via les trois émetteurs de stimulation.

9. Dispositif de stimulation par acupuncture porté au poignet selon la revendication 8, comprenant en outre :
une électrode de masse montée sur la face inférieure du boîtier, et
dans lequel les trois émetteurs de stimulation sont des électrodes configurées pour émettre un courant directement vers les points d'acupuncture H7, Lu9 et P6, et l'électrode de masse est configurée pour collecter le courant provenant de l'une ou de l'ensemble des trois électrodes après son passage par les points d'acupuncture H7, Lu9 ou P6, et renvoyer ledit courant vers l'alimentation en impulsions à tension variable.

10. Dispositif de stimulation par acupuncture porté au poignet selon la revendication 8 ou 9, comprenant en outre un dispositif de photopléthysmographie (PPG) configuré pour mesurer au moins la variabilité de la fréquence cardiaque (VFC) et la fréquence cardiaque (FC).

11. Dispositif de stimulation par acupuncture porté au poignet selon la revendication 10, dans lequel, lorsque le dispositif PPG détecte une diminution du niveau de HRV, le dispositif de stimulation par acupuncture porté au poignet est activé pendant une durée prédéfinie.

12. Dispositif de stimulation par acupuncture porté au poignet selon l'une quelconque des revendications 8 à 11, comprenant en outre une communication à courte portée avec un téléphone mobile, dans lequel le téléphone mobile exécute un programme d'application stocké utilisant ladite communication à courte portée.

13. Dispositif de stimulation par acupuncture porté au poignet selon l'une quelconque des revendications 8 à 12, dans lequel les trois émetteurs de stimulation sont des électrodes configurées pour émettre un courant en tant que stimulation, des émetteurs de lumière configurés pour émettre une lumière dirigée en tant que stimulation, des émetteurs haptiques configurés pour émettre un son ou une vibration en tant que stimulation, ou des électroaimants configurés pour émettre des champs magnétiques en tant que stimulation.

14. Dispositif de stimulation par acupuncture porté au poignet selon l'une quelconque des revendications 8 à 13, dans lequel les trois émetteurs de stimulation sont une combinaison de deux ou plusieurs des éléments suivants : des électrodes configurées pour émettre un courant en tant que stimulation, des émetteurs de lumière configurés pour émettre une lumière dirigée en tant que stimulation, des émetteurs haptiques configurés pour émettre un son ou une vibration en tant que stimulation, ou des électroaimants configurés pour émettre des champs magnétiques en tant que stimulation.
